**Europäisches Patentamt**

**European Patent Office**

**Office européen des brevets**

(19)

(11) Veröffentlichungsnummer : **0 012 295**
**B1**

(12) **EUROPÄISCHE PATENTSCHRIFT**

(45) Veröffentlichungstag der Patentschrift :
05.10.83

(51) Int. Cl.³ : **A 61 F 13/16, A 41 B 13/02**

(21) Anmeldenummer : **79104807.7**

(22) Anmeldetag : **01.12.79**

(54) **Hygienevorlage.**

(30) Priorität : **09.12.78 DE 2853243**

(43) Veröffentlichungstag der Anmeldung :
**25.06.80 Patentblatt 80/13**

(45) Bekanntmachung des Hinweises auf die Patenterteilung : **05.10.83 Patentblatt 83/40**

(84) Benannte Vertragsstaaten :
**BE CH DE FR IT LU NL SE**

(56) Entgegenhaltungen :
**BE A 702 524**
**BE A 815 446**
**CH A 464 440**
**DE A 1 924 813**
**DE C 547 661**
**DE U 1 879 601**
**FR A 1 229 858**
**FR A 1 501 868**
**FR A 2 374 890**
**GB A 1 271 520**
**US A 2 693 806**
**US A 3 343 543**
**US A 3 344 789**
**US A 3 424 163**
**Kunststoff-Taschenbuch, 20. Auflage, 1977, Ste.**
**130, 141, 172, 173**

(73) Patentinhaber : **Henkel Kommanditgesellschaft auf Aktien**
**Postfach 1100 Henkelstrasse 67**
**D-4000 Düsseldorf 1 (DE)**

(72) Erfinder : **Wiegner, Georg**
**Breslauer Strasse 35**
**D-4060 Viersen 11 (DE)**

## Hygienevorlage

Die Erfindung betrifft eine Hygienevorlage zum Aufnehmen von Körperflüssigkeit aus einer Flüssigkeitsaustrittsöffnung des Körpers mit einer saugfähigen Einlage aus hydrophilen, mit mindestens einer Umhüllung aus Vliesstoff, Gewebe oder Tissue versehenen Fasermaterialien, wobei wenigstens eine Schicht der äußeren Umhüllung, und zwar die innenliegende Schicht, flüssigkeitsundurchlässig ist, und wobei die Umhüllung an der der Flüssigkeitsaustrittsöffnung zuzuwendenden Seite eine definierte flüssigkeitsdurchlässige Saugzone aufweist und wobei im Kern der Einlage ein saugfähiges Papierband angeordnet ist, das allseitig von dem hydrophilen Fasermaterial umhüllt ist.

Eine Hygienevorlage dieser Gattung ist beispielsweise bekannt aus der US-A-3 343 543. Eine ähnlich aufgebaute Hygienevorlage ist fernerhin aus der DE-A-19 24 813 bekannt, wobei die Hülle aus einem formbeständigen hydrophoben Material in Gestalt eines weichen flüssigkeitsdurchlässigen Schaumes aus einem organischen synthetischen Material hergestellt und von dem absorbierenden Kern durch einen flüssigkeitsdichten Film getrennt ist, der um die Längskanten der Öffnung auf die Außenseite der Hülle zurückgefaltet und an dieser befestigt ist.

Schließlich war es auch schon aus der CH-A-464 440 bekannt, bei einer Damenbinde die Hülle aus einem hydrophobierten Faserstoff herzustellen, sowie die Hülle dergestalt auszuführen, daß diese auf der vom Körper abgewandten Seite und an den Kanten flüssigkeitsdicht ist.

Solche Hygienevorlagen werden in verschiedenen Ausführungen verwendet, z. B. in der Baby-Hygiene als Windeln oder Höschen, in der Menstruations-Hygiene als Binden oder Slip-Einlagen, in der Medizin als Wundsekretpackungen oder Urinalvorlagen. Bei diesen Hygienevorlagen ist von großer Bedeutung, daß sie bequem im Tragen, angenehm auf der Haut und nicht zu dick auftragend gestaltet sind um möglichst frei von Geruchsentwicklung. Da es sich um Wegwerfartikel handelt, die relativ häufig verwendet werden, spielen auch der Preis und die Vernichtbarkeit eine Rolle.

Der Verwender ist häufig gezwungen, über mehrere Tage (Binden) oder gar einen langen Zeitraum (Windeln, Slipeinlagen, Urinalvorlagen) Tag und Nacht solche Vorlagen zu tragen. Die Benutzung der Hygienevorlage führt daher häufig zu erheblichen Belastungen, wie Hautirritationen, Geruchsentwicklung oder Beschmutzung der Wäsche und Kleidung. Des weiteren fühlen sich die Verwender oft gehemmt, psychisch belastet durch die Tatsache, daß ihre Umwelt Kenntnis von der Verwendung von Hygienevorlagen erhält, z. B. dadurch, daß wegen der Größe der Vorlage diese durch die Kleidung sichtbar ist oder das Tragen von leichter Kleidung, insbesondere von Badezeug, unmöglich wird. An warmen Sommertagen kommt es häufig zu Schweißbildungen im Bereich der Vorlagen, die sehr lästig sind. Angstzustände können geradezu die Vorstellung auslösen, daß die Vorlage nicht mit Sicherheit die Körperflüssigkeit bindet und es möglicherweise zu einem für jedermann sichtbaren Durchsickern kommt.

Alles in allem ist trotz in der Vergangenheit erzielter erheblicher Fortschritte beim Gestalten von Hygienevorlagen bis heute noch kein umfassend befriedigendes Ergebnis erzielt worden. Ein solches würde voraussetzen, daß ein Produkt folgende Forderungen optimal und zugleich erfüllt :

a) Sicherheit vor Durchsickern ;

b) keine wesentlich wahrnehmbare Geruchsbildung ;

c) angenehmes Tragen, keine Hautirritationen ;

d) kleines Volumen und demgemäß auch bei leichter Kleidung nicht sichtbar zu tragen ;

e) wirtschaftlich herzustellen und daher preiswert.

Versuche, vorstehende Anforderungen an eine Hygienevorlage zu erfüllen, führten in der Vergangenheit in bezug auf Forderung a) bei zahlreichen Produkten zu voluminösen Ausführungen, die zwar eine ausreichende Saugfähigkeit auf weisen, aber weder die Forderung d) noch e) erfüllen.

Es sind auch schon Folien auf der körperabgewandten Seite der Vorlage eingesetzt worden, um ein Durchsickern zu verhindern. Dadurch werden jedoch die Forderungen nach angenehmer Tragbarkeit und kleinem Volumen (b und c) nicht erfüllt und der Flüssigkeitskontakt mit der Haut keineswegs ausgeschlossen. Das kommt daher, daß bei außen auf der Umhüllung angebrachten Folien das Durchsickern, z. B. bei Binden oder Windeln, nicht mit Sicherheit verhütet ist, da sich die Position der Hygienevorlage zwischen den Schenkeln den anatomischen Bedingungen anpaßt und demgemäß die Ränder der Vorlage mehr oder weniger Kontakt zu Wäsche und Kleidung bekommen. Dieser Kontakt wird durch Geh- oder Strampelbewegungen noch verstärkt, so daß es tendenziell zu einem Verrutschen der Folie und damit zu einem Ausquetschen von Flüssigkeit kommt. Darüber hinaus erzeugen außen auf der Umhüllung angebrachte Folien häufig unerwünschte Rascheleffekte.

Zum Verhindern des Kontaktes der Flüssigkeit mit der Haut oder der Kleidung sind Hygienevorlagen mit einer hydrophoben Umhüllung hergestellt worden. Diese Maßnahme bringt jedoch wenig Erfolg, da hydrophobe Umhüllungen keine flüssigkeitsdichte Vorlage ergeben und durch Körperbewegungen die zunächst gespeicherten Flüssigkeiten fast ungehindert ausgequetscht werden. Durch hydrophobe Umhüllungen kann lediglich erreicht werden, daß die Flüssigkeit nicht wie bei hydrophilen Umhüllungen dochtartig um den äußeren Umfang der Hygienevorlage herumgesaugt wird.

Der Erfindung liegt die Aufgabe zugrunde, eine Hygienevorlage eingangs genannter Art zu schaffen, die die vorgenannten Hauptforderungen a) bis e) gleichzeitig und optimal erfüllt, die sowohl in Anwendung als auch in Anbringung bzw. Fixierung am Körper einfach zu handhaben ist und die einfach und billig herzustellen ist.

Diese Aufgabe wird erfindungsgemäß im wesentlichen durch eine Hygienevorlage gemäß Anspruch 1 gelöst.

Daß die neue Hygienevorlage die gestellte Aufgabe in so hervorragender Weise erfüllt und allen Anforderungen gerecht wird, ist im Zusammenwirken aller Merkmale bedingt. Die definierte Saugzone im Bereich der Flüssigkeitsaustrittsöffnung des Körpers läßt die abgesonderte Flüssigkeit genau dort eintreten, wo sie schnell und vollständig adsorbiert werden soll. Diese gezielte Aufnahme wird noch unterstützt durch die Einlage aus hochsaugfähigen Materialien, die geradezu eine Saug- und Dochtwirkung durch die saugfähige Zone hindurch entfaltet und für ein Festhalten der Flüssigkeit im Innern der Hygienevorlage sorgt. Die flüssigkeitsundurchlässige Umhüllung in Form des Verbundmaterials im gesamten übrigen Bereich der Hygienevorlage verhindert andererseits ein Wiederaustreten der Flüssigkeit bei längerem Gebrauch und heftigen Bewegungen. Hinsichtlich der einzelnen Anforderungen ergibt sich :

Die erfindungsgemäße Hygienevorlage bietet zunächst Sicherheit vor Durchsickern (Forderung a). Das wird dadurch erreicht, daß die Vorlage wegen der flüssigkeitsundurchlässigen Ausrüstung der Umhüllung und wegen der hochsaugfähigen Einlage die in den speziell geschaffenen relativ kleinen, definierten Saugzonen aufgesogenen Körperflüssigkeiten wie in einer Art Feuchtetresor festhält und nicht an umgebende, mit der Vorlage in Kontakt stehende Haut- oder Kleidungspartien abgibt.

Die Geruchsbildung (Forderung b) wird bei der neuen Vorlage weitgehend durch den feuchte- und geruchsdichten Abschluß der aufgesogenen Flüssigkeiten im Innern der Hygienevorlage verhindert.

Die neue Vorlage ist ferner angenehm im Tragen und bedeutet keine Gefahr von Hautirritationen (Forderung c). Durch das weitgehende Abschirmen von Haut und Wäsche gegenüber einem Flüssigkeitskontakt werden Hautirritationen verhindert. Die angenehm weiche Beschaffenheit des Trägermaterials der äußeren Umhüllung bleibt auch im Verbundmaterial voll erhalten, da die undurchlässige Beschichtung auf der Innenseite der Vorlage angeordnet ist.

Die Vorlage ist ferner klein und kann auch bei leichter Kleidung getragen werden (Forderung d). Da die hochsaugfähige Einlage im Kern der Hygienevorlage zu einem hohen Prozentsatz ausgenutzt wird, kann die Vorlage wesentlich dünner und kleiner als bisher üblich ausgebildet werden, ohne daß die Gefahr eines Durchfeuchtens besteht.

Die Einlage weist im Kern ein bevorzugt ein- oder mehrfach gefaltetes Tissuepapier auf, auf das vor dem Falten hochsaugfähige Polymere in Granulat- oder Faserform aufgebracht sind. Diese hochsaugfähigen Polymere sind als solche vorbekannt. Sie vermögen das Hundertfache ihres Gewichtes ohne Volumenvergrößerung an Flüssigkeit zu binden. Es kommen insbesondere zwei Stoffgruppen dafür in Betracht, nämlich vernetzte Carboxymethylcellulose und Polyacrylate. Letztere sind deshalb ganz besonders bevorzugt, weil sie auch für Blut ein ausgezeichnetes Adsorptionsvermögen aufweisen. Das Tissuepapier ist sehr gut saugfähig und entfaltet damit eine Dochtwirkung und gibt die aufzunehmende Flüssigkeit gut an die hochsaugfähigen Polymere weiter. Die hochsaugfähigen Polymere werden zweckmäßig durch mehrfaches Falten des Tissuepapiers eingeschlossen und in diesem festgehalten.

Besonders vorteilhaft ist es, dem für die hochsaugfähigen Polymere als Trägermaterial vorgesehenen, besonders geeigneten Tissuepapier auf einem Kalander ein Waffelmuster zu geben und damit eine Vielzahl kleiner Taschen zu schaffen, in denen das hochsaugfähige Polymere, insbesondere wenn es als Granulat eingesetzt wird, untergebracht ist. Diese Prägung des Tissuepapiers kann vor dem Aufbringen der hochsaugfähigen Polymere erfolgen. Bevorzugt ist aber eine Arbeitsweise, bei der die hochsaugfähigen Polymere auf das Tissuepapierband mindestens der zweifachen Breite der Einlage aufgebracht werden, dann die freien Randbereiche umgefaltet und die hochsaugfähigen Polymere so eingeschlossen werden und erst daran anschließend durch kalandrieren das Waffelmuster eingeprägt wird. Das Einprägen schafft dann die Taschen, in denen sich das hochsaugfähige Polymere bevorzugt ansammelt, und verbindet gleichzeitig die zwei oder drei Lagen Tissuepapierband, wie sie durch das Umfalten erhalten werden, genügend fest miteinander. Da die hochsaugfähigen Polymere als Fasern oder feines Granulat lose aneinanderliegen und untereinander keine Kapillarität entwickeln, übernimmt das Tissuepapier mit seiner Dochtwirkung die Drainage für die Flüssigkeit zu den Speichertaschen mit hochsaugfähigen Polymeren. Diese wiederum nehmen zwar bis zum Hundertfachen ihres Gewichts an Flüssigkeit auf, ohne daß sich aber ihr Volumen nennenswert vergrößert und ohne daß diese die Flüssigkeit wieder abgeben — auch nicht bei Quetscheinwirkung.

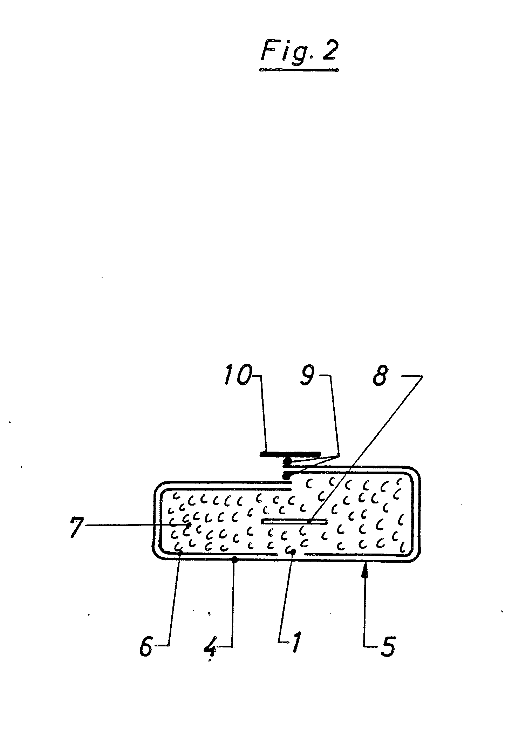

Die Anordnung dieses die hochsaugfähigen Polymere enthaltenden Tissuepapierbandes im Kern der Vorlage, wie in Fig. 2, 3 und 4 dargestellt, garantiert den Verbleib der Flüssigkeit im Innern der Vorlage und vermeidet damit Geruchsbildung und Durchfeuchten, und ermöglicht darüber hinaus eine besonders dünne und kleine Ausführung der Hygienevorlage.

Schließlich ist die Hygienevorlage wirtschaftlich herzustellen und damit preiswert (Forderung e). Wegen der geringen Größe und des entsprechend geringen Materialeinsatzes sowie wegen der besonderen Konstruktion der äußeren Umhüllung ist ein wirtschaftliches Herstellen auf handelsüblichen Maschinen möglich. Die neue Vorlage kann daher sehr preisgünstig angeboten werden.

Das die äußere Umhüllung bildende Verbundmaterial hat folgenden Aufbau :

Es besteht außenseitig aus einem flüssigkeitsdurchlässigen Trägermaterial aus Gewebe, Vlies oder Tissue und ist damit ausgesprochen hautfreundlich und angenehm im Tragen. Innenseitig besteht es mit Ausnahme der definierten Saugzone aus einer flüssigkeitsundurchlässigen Schicht, die den Durchtritt der Flüssigkeit nach außen verhindert. Dabei ist es ganz wesentlich, daß das Trägermaterial und die flüssigkeitsundurchlässige Schicht zu einem Verbundmaterial vereinigt sind, also nicht wie bisher lose aneinanderliegen und gegebenenfalls in Randzonen der Hygienevorlage miteinander verbunden sind. Bei den unvermeidlichen Körperbewegungen führte das immer wieder zum Zwischentreten der Flüssigkeit zwischen die einzelnen Lagen und zum Ausquetschen der Flüssigkeit.

Bevorzugt ist auch das Trägermaterial hydrophob ausgerüstet, weil es im Bereich der definierten Saugzone direkt mit der Flüssigkeit in Berührung kommt und diese nicht aufsaugen soll. Hydrophob bedeutet dabei aber lediglich, daß es keine oder nur wenig Flüssigkeit aufnimmt. Das Trägermaterial läßt aber trotz seiner hydrophoben Ausrüstung unter Körperkontakt die aufzunehmende Flüssigkeit gut hindurchtreten und von der Füllung aufnehmen.

Die innenseitige flüssigkeitsundurchlässige Schicht der äußeren Umhüllung, also das Verbundmaterial, ist anspruchsgemäß durch Auflaminieren, Aufkalandrieren, Aufextrudieren, Beschichten, Bondieren oder einseitiges Imprägnieren, Aufdrucken oder Aufstreuen erzeugt. Unter Bondieren versteht man dabei das Auftragen eines Bindemittels, beispielsweise in Form von Kunststoff- oder Kautschuklatices. Beim einseitigen Imprägnieren läßt man das Trägermaterial die Imprägnierflüssigkeit nur an der Oberfläche des Imprägnierbades berühren, sodaß keine Durch- und Durch-Imprägnierung erfolgt. Ganz bevorzugt ist seiner Einfachheit und Schnelligkeit wegen der Auftrag der flüssigkeitsundurchlässigen Schicht durch Bedrucken.

Stofflich besteht die flüssigkeitsundurchlässige Schicht bevorzugt aus Kunststoffolien, Thermoplastpulvern, Thermoplastpasten, Heiß- oder Kaltklebstoffe, Polyurethan, Kautschuk, chemischen Bindern, Imprägniermitteln oder zeitlich begrenzt wasserbeständigen Polyvinylalkoholpolymerisaten, wie sie aus Polyvinylacetaten durch ganz bestimmten Verseifungsgrad hergestellt werden. Die zeitlich begrenzte Wasserbeständigkeit, die hiermit erreicht wird, führt dazu, daß so ausgerüstete Hygienevorlagen mit viel Wasser hinweggespült werden können, d. h. toilettierbar sind.

Bewährt haben sich auch farbige Beschichtungen, Imprägnierungen bzw. Bondierungen und Aufdrucke, weil dadurch die undurchlässigen Bereiche von den durchlässigen Bereichen der Umhüllung deutlich abgesetzt sind. Die farbige Ausgestaltung der flüssigkeitsundurchlässigen Schicht hat den Vorteil, daß dabei auch Aufdrucke wie z. B. Warenzeichen mit angebracht werden können.

Bei der Hygienevorlage zur Aufnahme von Körperflüssigkeit kommt der definierten Saugzone und ihrer Ausgestaltung Bedeutung zu. Dabei ist in bekannter Weise aus der äußeren Umhüllung aus flüssigkeitsundurchlässigem Material eine Art Fenster — die sogenannte definierte Saugzone ausgespart, indem dieser Bereich von der flüssigkeitsundurchlässigen Beschichtung ausgespart ist und damit dort die Flüssigkeit durchtreten kann. Diese flüssigkeitsdurchlässige Saugzone kann eine der zu beschirmenden Flüssigkeitsaustrittsöffnung angepaßte Form haben. Die Sicherheit gegen ein Durchsickern oder Vorbeilaufen der aufzufangenden Flüssigkeit wird jedoch unter Umständen erhöht, wenn die Saugzone nach allen Seiten gerichtete Saugarme aufweist. Die Saugzone selbst kann zur Verbesserung der Drainage und zur Weiterleitung zur hochsaugfähigen Einlage hin perforiert oder genadelt sein.

Die Einlage der erfindungsgemäßen Hygienevorlage im Inneren der Füllung besteht aus einem ein- oder mehrfach gefalteten Tissuepapierband, auf das vor dem Falten die hochsaugfähigen Polymere aufgebracht sind, sei es in Faserform oder bevorzugt als Granulat.

Ganz bevorzugt ist dabei eine Ausführungsform für die Einlage, bei der in das ein- oder mehrfach gefaltete Tissuepapierband mit einem Kalander ein Waffelmuster eingeprägt ist, so daß bei hochsaugfähigen Polymeren in Granulatform diese in einer Vielzahl kleiner Taschen gespeichert sind und damit sehr gleichmäßig und auch unverrückbar über die gesamte Länge und Breite der Einlage festgelegt sind. Dieses Einbringen des Waffelmusters kann bereits vor der Zugabe der hochsaugfähigen Polymeren erfolgen. Bevorzugt werden aber die hochsaugfähigen Polymere vorher auf das Tissuepapierband aufgebracht, dieses dann ein-, bevorzugt aber zweimal umgefaltet und erst dann durch Kalandrieren das Waffelmuster eingeprägt. Das hat den Vorteil, daß sich dabei das Granulat in den so gebildeten Taschen sammelt, gleichzeitig aber auch die Lagen untereinander verbunden werden.

Es ist aber auch möglich und durchaus zweckmäßig, die hochsaugfähigen Polymere zusätzlich auf dem Tissuepapierband zu verankern, beispielsweise durch Klebstoff oder durch vorsichtige Naßbehandlung des Tissuepapierbandes. Die dazu erforderliche Flüssigkeit ist äußerst gering im Vergleich zu den großen Mengen Flüssigkeit, die die hochsaugfähigen Polymere aufzunehmen in der Lage sind, so daß ihre Funktionsfähigkeit durch eine solche Naßbehandlung in keiner nennenswerten Weise beeinträchtigt wird.

Daß die hochsaugfähigen Polymere auf ein Tissuepapierband aufgebracht werden, ist darin begründet, daß Tissue eine ganz hervorragende Verteilerwirkung für Flüssigkeiten hat und eine große Dochtwirkung entfaltet und so die Flüssigkeit hervorragend an das hochsaugfähige polymere Material heranträgt.

Der besondere Vorzug dieser erfindungsgemäßen Ausgestaltung der Hygienevorlage, insbesondere der äußeren Umhüllung und der Einlage, besteht zum weiteren in der außerordentlichen Wirtschaftlichkeit der Verarbeitung und Herstellung. Alle bisher bekannten Verfahren und Maschinen zur Herstellung von Hygienevorlagen verarbeiten alle Bestandteile der Vorlage in einem Arbeitsgang auf der Maschine. Insbesondere werden die verschiedenen Lagen der Umhüllung der Füllung von Rollen zugeführt und müssen durch genaues Positionieren, Umlegen, Überlappen, Verkleben etc. um die Hygienevorlage herum angeordnet werden. Dabei tendieren Hüllmaterialien mit relativ hoher Oberflächenglätte zum Verrutschen. Es ist außerdem einleuchtend, daß je mehr einzelne Bestandteile einer Hygienevorlage auf einer Maschine kombiniert werden müssen, desto komplizierter die Maschinenführung und desto aufwendiger die Arbeitsbelastung durch Einfädeln, Rollenwechsel, Lagerhaltung etc. sich darstellt.

Diese Mängel werden durch den erfindungsgemäßen Hüllstoff weitgehend vermieden. Durch die Tatsache, daß die äußere Umhüllung als Verbundmaterial ausgeführt wird, ergibt sich die technische Möglichkeit, dieses Verbundmaterial völlig unabhängig von der eigentlichen Produktionsmaschine für Hygienevorlagen in einer Vorstufe herzustellen.

Diese Vorstufe läßt sich in der Arbeitsweise räumlich und zeitlich unabhängig von der späteren Hauptstufe trennen. So werden bei dem Herstellungsverfahren nach rationellsten Fertigungstechniken in großen Maschinenbreiten Materialbahnen gefertigt, die je nach unterschiedlicher Vorlagenbreite später bedarfsweise aufgeschnitten werden. Die so aufgeschnittenen Rollen können nun auf der Hygienevorlagen-Maschine mit besonders hohen Fertigungsgeschwindigkeiten verarbeitet werden, da sie :

1. durch den Verbund stabiler sind als vergleichbar mehrbahnig zugeführte Materialien. Hierbei ist als üblich Vliesstoff + Tissue + Kunststoffolie anzusehen. Besonders die nur 15-20 μ starke Kunststoffolie tendiert durch statische Aufladung, glatte Oberfläche und Instabilität zu Schwierigkeiten in der Verarbeitung. Aufkaschiert auf das Trägermaterial, also als Verbundmaterial, werden diese Schwierigkeiten vermieden.

2. wiederum bedingt durch den Einsatz eines Verbundmaterials als äußere Umhüllung durch geringere Anzahl von Arbeitsstationen, leichtere Bedienung, weniger Rollenwechsel etc. eine höhere Arbeitsintensität erlauben.

Anhand der schematischen Darstellung von Ausführungsbeispielen werden weitere Einzelheiten der Erfindung erläutert. Es zeigen :

Figur 1 die Draufsicht auf eine Hygienevorlage mit mittig, gerade verlaufender Saugzone ;

Figuren 2 und 3 Querschnitte durch zwei verschiedene Ausführungsbeispiele ;

Figur 4 ein Längsschnitt durch die Vorlage nach Fig. 1 und 2 ;

Figur 5 eine Vorlage in der Draufsicht mit einer Saugzone mit unregelmäßig verlaufenden Längslinien ; und

Figur 6 eine Vorlage in der Draufsicht mit einer Saugzone mit sternförmig angeordneten Saugarmen.

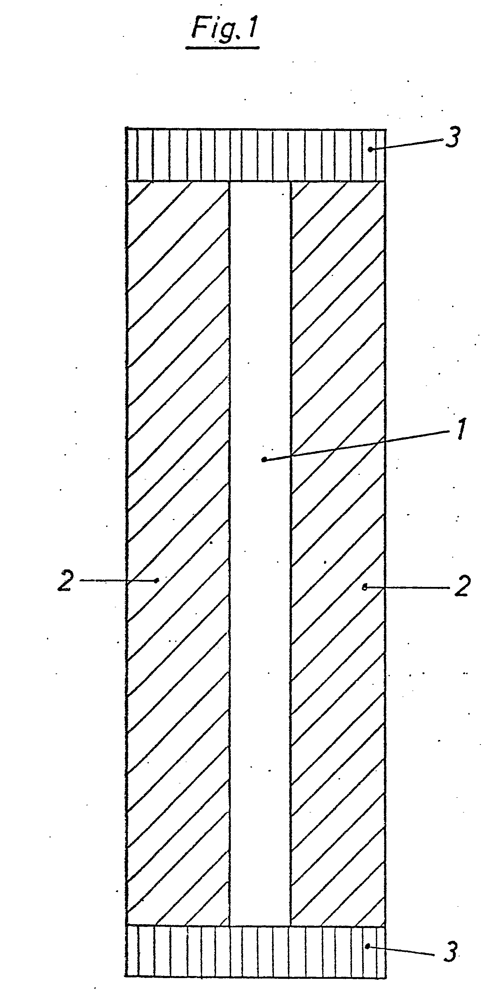

Die Zeichnung gemäß Fig. 1 zeigt eine Vorlage in der Draufsicht mit gerade und parallel zur Längserstreckung der Vorlage verlaufender Saugzone 1, beiderseits der Saugzone 1 angeordneten flüssigkeitsundurchlässigen Bereichen 2 und an den Längsenden vorgesehenen Verschlußzonen 3.

Fig. 2 zeigt einen Querschnitt durch eine Vorlage gemäß Fig. 1. Die Saugzone 1 liegt hier auf der Unterseite der Vorlage in dem Bereich, in dem die auf der Innenseite des Basismaterials 4 der insgesamt mit 5 bezeichneten Umhüllung vorgesehene flüssigkeitsundurchlässige Beschichtung 6 eine Öffnung aufweist. Die Umhüllung 5 ist im wesentlichen mit hydrophilem Fasermaterial 7 ausgefüllt. Im Zentrum der Umhüllung 5 befindet sich ein hochsaugfähiges Tissuepapierband 8. Im Ausführungsbeispiel ist auf der der Saugzone 1 gegenüberliegenden Seite der Vorlage eine aus Klebstoff 9, zum Beispiel Hotmelt, und Silikonpapier 10 gebildete Klebenaht vorgesehen.

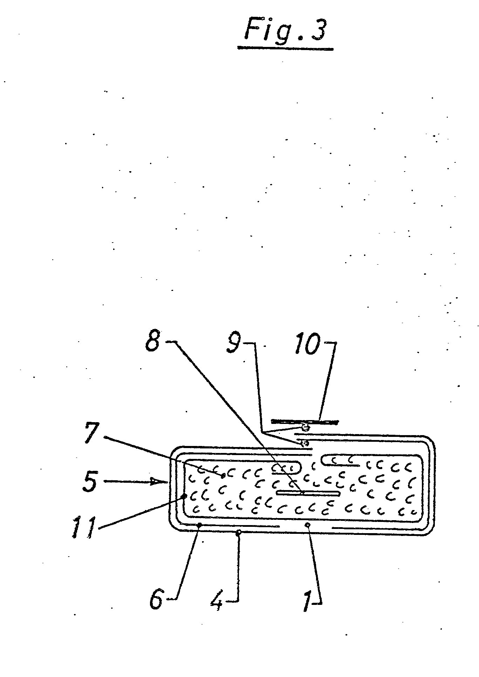

Im wesentlichen genauso wie die Vorlage gemäß Fig. 2 ist diejenige nach Fig. 3 aufgebaut. Der wesentliche Unterschied besteht jedoch darin, daß das hydrophile Fasermaterial 7 innerhalb der Umhüllung 5 ganz von Tissue 11 umgeben ist.

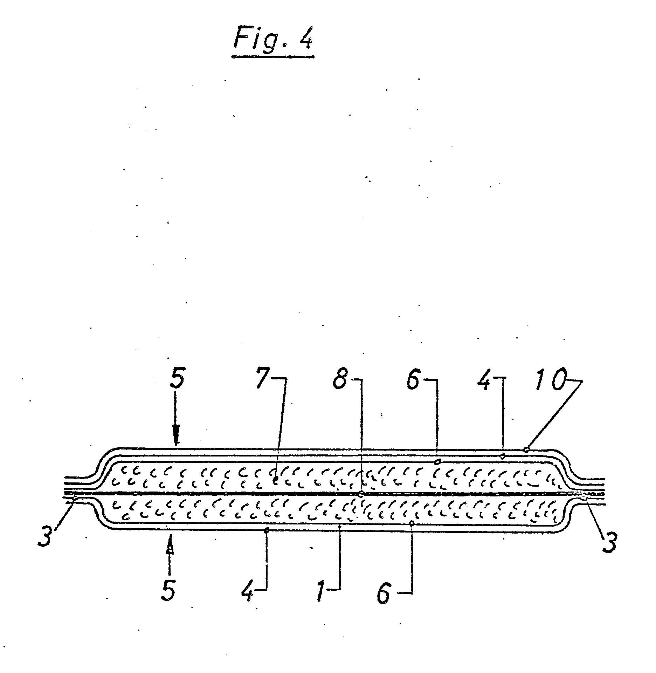

Figur 4 zeigt den Längsschnitt durch eine Vorlage gemäß Fig. 1 und 2, wobei im wesentlichen die Bezugszeichen von Fig. 2 verwendet worden sind.

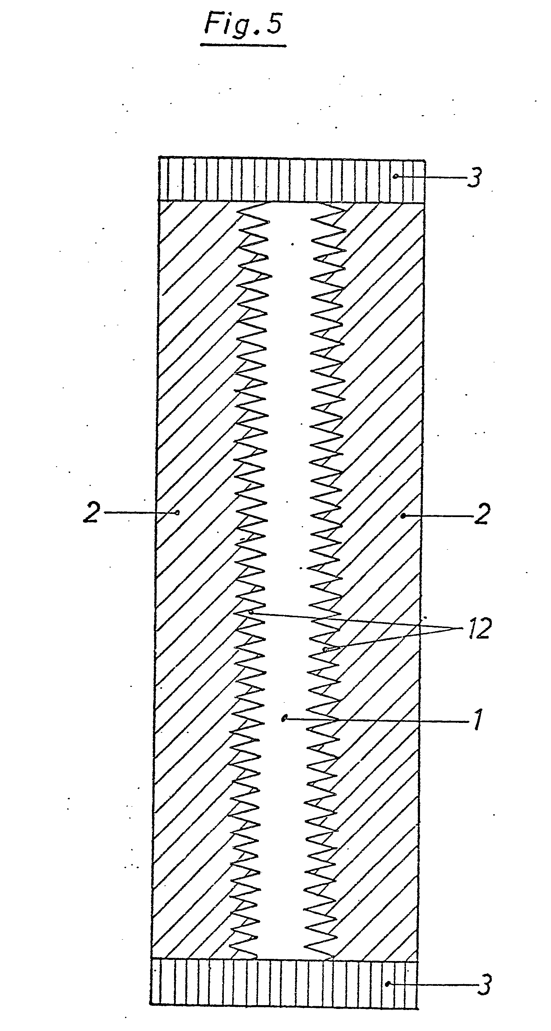

Die Vorlage gemäß Fig. 5 unterscheidet sich von derjenigen nach Fig. 1 vor allem dadurch, daß die Saugzone 1 nicht gerade Grenzen zu den flüssigkeitsundurchlässigen Bereichen 2 sondern in Form von unregelmäßig verlaufenden Längslinien 12 aufweist.

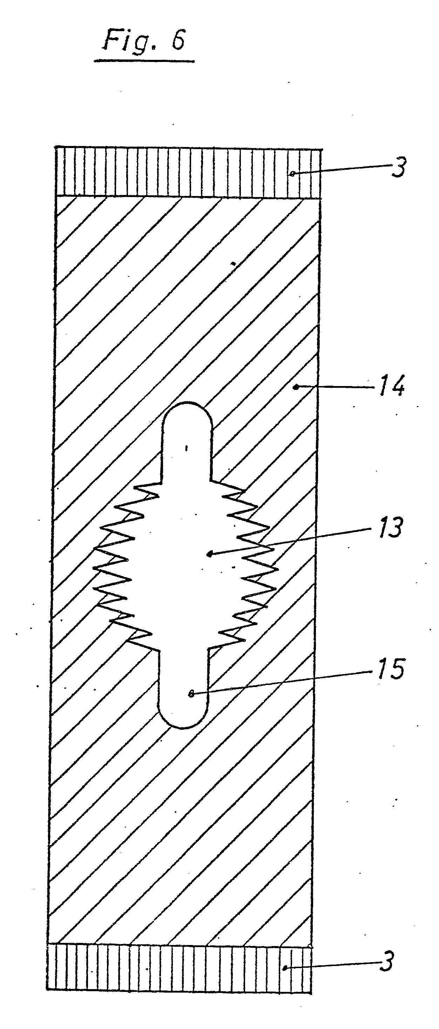

Bei der Vorlage gemäß Fig. 6 liegt die Saugzone im wesentlichen zentral innerhalb eines flüssigkeitsundurchlässigen Bereichs 14. Die Saugzone 13 kann dabei sternförmig angeordnete Saugarme 15 besitzen die sich teilweise in Längsrichtung der Vorlage und teilsweise unregelmäßig in Querrichtung (ähnlich den Längslinien 12 von Fig. 5) erstrecken.

**Ansprüche**

1. Hygienevorlage zum Aufnehmen von Körperflüssigkeit aus einer Flüssigkeitsaustrittsöffnung des Körpers mit einer saugfähigen Einlage aus hydrophilen, mit mindestens einer Umhüllung aus Vliesstoff, Gewebe oder Tissue versehenen Fasermaterialien (7), wobei wenigstens eine Shicht (6) der äußeren Umhüllung (5), und zwar eine innenliegende Schicht (6), flüssigkeitsundurchlässig ist, und wobei die Umhüllung (5) an der der Flüssigkeitsaustrittsöffnung zuzuwendenden Seite eine definierte flüssigkeitsdurchlässige Saugzone (1, 13) aufweist und wobei im Kern der Einlage ein saugfähiges Papierband (8) angeordnet ist, das allseitig von dem hydrophilen Fasermaterial (7) umhüllt ist, dadurch gekennzeichnet, daß der flüssigkeitsundurchlässige Bereich (2) des Basismaterials der äußeren Umhüllung (5) als durch Auflaminieren, Aufkalandrieren, Aufextrudieren, Bondieren oder Imprägnieren bzw. durch Aufbringen im Druckverfahren oder Pulverstreuverfahren hergestellte und auf die äußerste Lage der Umhüllung (5) aus Vliesstoff oder Tissue direkt aufgebrachte Beschichtung (6) ausgebildet ist, wobei die flüssigkeitsundurchlässige Schicht innen liegt und wobei in diesem so hergestellten Verbundmaterial dadurch eine definierte Saugzone (1, 13) geschaffen ist, daß von dieser flüssigkeitsundurchlässigen Beschichtung (6) die definierte Saugzone (1, 13) ausgespart ist, und daß das saugfähige Papier (8) im Kern ein hochsaugfähiges Tissuepapier ist, auf das hochsaugfähige Polymere oder hydrophile Fasern aufgebracht sind.

2. Vorlage nach Anspruch 1, gekennzeichnet durch ein ein- oder mehrfach gefaltetes Tissuepapier (8) mit einem kalandrierten Waffelmuster mit einer Vielzahl von rieselfähiges, hydrophiles Fasermaterial oder Polymere gespeichert enthaltenden kleinen Taschen.

3. Vorlage nach Anspruch 1 oder 2, dadurch gekennzeichnet, daß das hydrophile Fasermaterial (7) bzw. die Polymere vor dem Falten und Kalandrieren mit Hilfe von Klebstoff auf dem Tissuepapier (8) verankert sind.

4. Vorlage nach einem oder mehreren der Ansprüche 1 bis 3, gekennzeichnet durch durch Naßbehandeln auf dem Tissuepapier (8) aufgebrachte hochsaugfähige hydrophile Polymere (7).

5. Vorlage nach einem oder mehreren der Ansprüche 1 bis 4, dadurch gekennzeichnet, daß die flüssigkeitsdurchlässige Saugzone (1) perforiert oder genadelt ist.

## Claims

1. Sanitary towel, for the absorbing of body fluid from a fluid exit opening of the body, with an absorbent insert of hydrophilic fibre materials (7) provided with at least one sheathing of non-woven textiles, woven fabric or tissue, wherein at least one layer (6) of the outer sheathing (5), and namely an inward layer (6), is impermeable to fluid and wherein the sheathing (5) at the side to be turned towards to the fluid exit opening displays a defined suction zone (1, 13) permeable by fluid an wherein an absorbent paper tape (8), which is sheathed on all sides by the hydrophilic fibre material (7), is arranged in the core of the insert, characterised thereby, that the region (2), which is impermeable to fluid, of the base material of the outer sheathing (5) is formed as coating (6) produced by laminating-on, calendering-on, extruding-on, bonding or impregnating or by applying in the pressure process or powder-scattering and applied directly onto the outermost layer of the sheathing (5) of non-woven textiles or tissue, wherein the layer impermeable to liquid lies inside and wherein a defined suction zone (1, 13) is thereby created in this compound material thus produced, that the defined suction zone (1, 13) is hollowed out of this coating (6) impermeable to fluid and that the absorbent paper (8) in the core is a highly absorbent tissue paper, onto which highly absorbent polymers or hydrophilic fibres are applied.

2. Towel according to claim 1, characterised by a tissue paper (8) folded once or several times and with a calendered waffle pattern with a plurality of small pockets containing stored pourable hydrophilic fibre material or polymers.

3. Towel according to claim 1 or 2, characterised therbey, that the hydrophilic fibre material (7) or the polymers are anchored to the tissue paper (8) with the aid of adhesive substance before the folding and calendering.

4. Towel according to one or more of the claims 1 to 3, characterised by highly absorbent hydrophilic polymers (7) applied onto the tissue paper (8) by wet treatment.

5. Towel according to one or more of the claims 1 to 4, characterised thereby, that the suction zone (1) permeable by fluid is perforated or needle-pierced.

## Revendications

1. Serviette hygiénique destinée à absorber des humeurs venant d'un orifice de sortie de liquide du corps, comportant une couche intercalaire absorbante en matières fibreuses hydrophiles (7) munies d'au moins une enveloppe formée de lainage, de tissu ou de papier mousseline, au moins une couche (6) de l'enveloppe extérieure (5), à savoir une couche (6) située à l'intérieur, étant imperméable aux liquides et l'enveloppe (5) présentant, du côté destiné à être tourné vers l'orifice de sortie de liquide, une zone d'absorption définie (1, 13) perméable aux liquides, une bande de papier absorbante (8) étant disposée au cœur de la couche intercalaire et étant enveloppée de tous côtés par la matière fibreuse hydrophile (7), cette serviette étant caractérisée par le fait que la zone imperméable aux liquides (2) de la matière de base de l'enveloppe extérieure (5) est conçue sous forme d'un revêtement (6) appliqué directement sur la couche la plus extérieure de l'enveloppe (5) en lainage, tissu ou papier mousseline, réalisé par stratification, calandrage, extrusion en place, liaison ou imprégnation ou par application d'un procédé d'impression ou de dissémination de poudre, la couche imperméable aux liquides étant située à l'intérieur et une zone d'absorption déterminée (1, 13) étant par conséquent formée dans cette matière composite ainsi fabriquée, et que dans ce revêtement imperméable aux liquides (6) est ménagée la zone d'absorption (1,13), la bande de papier absorbant (8) située au cœur de la couche intercalaire étant constituée de papier mousseline très absorbant sur lequel sont appliquées des fibres polymères ou hydrophiles très absorbantes.

2. Serviette hygiénique selon la revendication 1, caractérisée par le fait que la bande de papier absorbant (8) sous forme de papier mousseline est pliée une ou plusieurs fois sur elle-même et présente un dessin gaufré calandré comportant de multiples petits creux contenant, à l'état emmagasiné, de la matière fibreuse hydrophile apte à l'écoulement ou des polymères.

3. Serviette hygiénique selon l'une ou l'autre des revendications 1 ou 2, caractérisée par le fait que la matière fibreuse hydrophile (7) ou les polymères ont été ancrés sur le papier mousseline (8) à l'aide d'un adhésif avant les opérations de pliage et de calandrage.

4. Serviette hygiénique selon l'une quelconque des revendications 1 à 3, caractérisée par le fait que des polymères hydrophiles (7) très absorbants sont appliqués sur le papier mousseline (8) par traitement humide.

5. Serviette hygiénique selon l'une quelconque des revendications 1 à 4, caractérisée par le fait que la zone d'absorption perméable aux liquides (1) est perforée ou percée de trous d'aiguille.

## Fig.1

_Fig. 2_

## Fig.3

## Fig. 4

## Fig.5

## Fig. 6